# EUROPEAN PATENT APPLICATION

(11) **EP 2 305 317 A1**
(43) Date of publication of application: **06.04.2011**
(21) Application number: 10194564.0
(22) Date of filing: 15.12.2004
(51) Int. Cl.: A61L 2/08

(54) **Terminal sterilization of prefilled containers**

(30) Priority: 15.12.2003 US 736489
(62) Divisional of application: 04814278.0
(71) Applicant: Becton Dickinson and Company, Franklin Lakes, New Jersey 07417 (US)
(72) Inventor: Zhao, Xia, Livingston, NJ 07417 (US); Ren, Jane, Warren, NJ 07059 (US); Odell, Robert, Franklin Lakes, NJ 07417 (US); Paterson, Patti, Branchville, NJ 07826 (US)
(74) Representative: von Kreisler Selting Werner

(57) **Abstract**

The invention relates to a sterilized article comprising:
a container made of a radiation stable polyolefin material; and
a medium contained within said container,

wherein said container containing said medium therein has been subjected to a gamma irradiation sterilization treatment after being filled with said medium.

## Description

### BACKGROUND OF THE INVENTION

### Field of the Invention

The invention is directed to sterilized medical devices. More particularly, the invention is directed to prefilled medical devices and containers which are stable for radiation sterilization and which meet the U.S. and European Pharmacopoeia requirements for medical devices that contain fluids for parenterally administered fluids.

### Description of Related Art

Prefilled medical devices, as the term is known in the art, are medical devices that are filled by the manufacturer at the time of assembly and are shipped in a ready-to-use condition to the healthcare provider. Prefilled medical devices have the advantage of convenience and ease of application with reduced risk of contamination of the contents of the device, such as a drug solution. Examples of prefilled medical devices include vials, cartridges, bottles, containers, and, most notably, syringes for parenteral administration of fluids such as pharmaceutical drugs. There are problems associated with providing a prefilled medical device, such as a syringe. For example, if the syringe is made of glass, issues arise with respect to breakage. If the syringe is made of a polymeric material, such as a polyolefin like polypropylene (PP), common sterilization procedures for medical devices can result in degradation of the sample, particularly with radiation sterilization procedures.

Terminal sterilization of medical devices is desired so as to reduce or to eliminate the risk of exposure to potential pathogens contained within the prefilled device. Various methods of sterilization of medical devices are known. For example, steam sterilization is commonly employed for sterilizing medical devices, which typically involves heating the device in a steam autoclave. Such steam sterilization, however, is time and labor consuming, and compromises the aesthetics of the product due to packaging degradation from the steam treatment. Radiation exposure is also commonly employed for sterilizing medical devices, in which the product is subjected to ionizing radiation, such as gamma irradiation.

For example, U.S. Patent No. 6,065,270 to Reinhard et al. discloses a method of producing a filled plastic syringe body by molding the syringe body, filling the syringe, sealing it, and sterilizing it, for example by autoclaving or high energy irradiation such as with E-beam radiation or gamma radiation. While this patent suggests that the solution contained within the syringe may dictate the sterilization method, it fails to teach how to maintain a safe solution within the syringe which meets pharmacopoeia requirements.

U.S. Patent No. 6,433,344 to Salisbury et al. discloses that gamma irradiation of polyolefin containers can result in weakened container integrity, leakage, increased gas permeability and an undesirable yellowing of the container, and that gamma radiation treatment inherently causes the generation of highly reactive species. The generation of such reactive species can alter the contents of the container being treated, thereby causing the contents of the container to fail the European and/or U.S. Pharmacopoeia requirements, such as pH standards (required to be between 4.5 and 7.0), UV absorbance levels (required to be below 0.2 at 220-340 nm), and the presence of hydrogen peroxide (H₂O₂) and other oxidizable substances (required to be below 1 × 10⁻⁴ mol/L or 3.4 ppm).

Despite the drawbacks of plastic syringes and the prefilling of medical devices, both are highly desirable since a syringe made from plastic provides durability and prefilling offers efficiency. Hence, there is a need to provide a prefilled device that meets the pharmacopoeia requirements prescribed for such devices.

### SUMMARY OF THE INVENTION

The present invention provides a method for inhibiting adverse reaction of the contents of a prefilled container during a radiation sterilization procedure, as well as a method of sterilizing a prefilled container. Such methods involve providing a container made of a material including a radiation stable polyolefin, and prefilling the container with a medium prior to subjecting the container to a gamma irradiation sterilization treatment. By using a radiation stable polyolefin material as the container, such as a polyolefin with a radiation stabilizer additive, and by prefilling the container prior to the gamma irradiation treatment, the container can be effectively sterilized, without adversely affecting its contents.

The medium prefilled within the container may be a therapeutic fluid or a non-therapeutic fluid. For example, the medium may be a saline solution, or may be a drug for parenteral administration to the body. After radiation sterilization, the medium should maintain specific properties within the pharmacopoeia requirements, such as a pH between about 4.5 and about 7.0, ultraviolet absorbance of less than about 0.2 at a wavelength between 220 and 340 nm, and less than about 3.4 ppm of oxidizable substances.

The container is constructed of a polyolefin material which is stable to gamma radiation, and desirably includes a radiation stabilizer such as a hindered piperidine stabilizer, and a liquid mobilizer. The container may further include other materials within its composition, such as additional polymer material, a clarifier and/or a nucleating agent. Desirably, the container is in the form of a syringe or a bag for intravenous fluid delivery.

Desirably, the container is sealed after being filled with the medium and prior to irradiation treatment. The container may further be enclosed within packaging, such as a blister package, prior to the irradiation treatment.

In a further embodiment, the present invention is directed to a sterilized article prepared through such a method. Such an article includes a container constructed of a polyolefin, a radiation stabilizer and a liquid mobilizer which includes a medium such as a medical fluid contained within the container, wherein the container containing the medium therein has been subjected to a radiation sterilization treatment after being filled with the medium.

In all embodiments described herein, the prefilled container need not be subjected to any other type of sterilization prior to the sterilization step in the method of the present invention, in which the prefilled container is subjected to gamma radiation. For example, there is no requirement that the container be manufactured or filled under sterile or aseptic conditions, as those terms are understood in the art. In fact, the present invention provides a benefit in that adverse reactions of the contents of a container filled prior to gamma radiation are prevented by the methods of the present invention, without the need for aseptic or sterile conditions during manufacture or fill.

### DESCRIPTION OF THE PREFERRED EMBODIMENTS

The invention is directed towards a method for providing a safe, prefilled medical device that has undergone terminal sterilization that meets U.S. and European Pharmacopoeia requirements. More particularly, the present invention relates to medical devices in the form of containers which are prefilled with a particular medium. For purposes of the present invention, containers are meant to include, but are not limited to, various medical devices and products, syringes, vials, cartridges, bottles and other containers of various sizes and shapes for containing a medium, in particular a fluid medium. The containers may be reuseable or disposable, and may have a medical, veterinary, or non-medical purpose. The present invention is particularly directed to syringes and bags for intravenous delivery of fluids to a patient. Also, the present invention is directed to containers for ophthalmic devices and lenses, such as disposable blister-type packages for packaging contact lenses in saline solution.

Generally speaking, it is well-known to construct containers useful as medical devices, and particularly syringes, out of polyolefin material. Polyolefin is particularly useful in such applications due to its ease of manufacture and inexpensive raw materials. For example, polypropylene (PP) has long been used as a material of choice for manufacturing syringes. However, as noted above, polyolefin materials such as PP are not particularly stable when subjected to ionizing radiation treatments, particularly gamma irradiation. Accordingly, such materials have traditionally not been used in applications in which prefilled devices undergo terminal sterilization through gamma radiation treatment.

It has been discovered through the present invention, however, that containers constructed of polyolefin can be sterilized through gamma irradiation treatment if the container structure is radiation stable, such as through the inclusion of a radiation stabilizer, and if the container is prefilled with a medium prior to the radiation treatment. In fact, it has unexpectedly been discovered that surprising results can be seen with respect to maintaining the integrity of the medium contained within a container through the use of radiation stable polyolefins in combination with gamma irradiation when the container is prefilled with the medium prior to such gamma irradation treatment.

The container structure of the present invention is radiation stable, that is, it maintains its integrity with respect to properties such as strength, leakage, gas permeability and color, when subjected to gamma irradiation. This may be accomplished by constructing the container out of a polyolefin composition which by its nature is radiation stable, or which includes additives in order to impart radiation stability to the polymer. For example, the container may be constructed of a cyclic olefin copolymer (COC), which by its nature is considered to be stable when exposed to gamma radiation typical of sterilization procedures.

More desirably, the container is constructed of a polyolefin composition which includes a radiation stabilizer to impart radiation stability to the container. Particularly useful are radiation stable polymeric compositions prepared in accordance with U.S. Patent Nos. 4,959,402 and 4,994,552, both of which are assigned to Becton, Dickinson and Company and both of which are incorporated in their entirety herein by reference.

For example, the polyolefin polymer may be described as basically linear, but may optionally contain side chains. They may be a homopolymer or a copolymer of an aliphatic monoolefin, preferably having about 2 to 6 carbon atoms. Desirably, the polyolefin may be generally selected from polyethylene, polypropylene, polymethylpentene, polytetrafluoroethylene and copolymers thereof. More desirably, the polyolefin is polypropylene.

It is desirable that the polyolefin of the composition be of a narrow molecular weight distribution. The molecular weight distribution of a polymer is defined by the ratio of the weight average molecular weight and the number average molecular weight wherein the minimum possible ratio of 1.0 defines the polymer having all the chains the same size. Suitable polyolefins for the composition of the invention may have a number average molecular weight of about 10,000 to 400,000, desirably 30,000 to 50,000 and a ratio of from 1 to 9 desirably about 2 to 6. More desirably, the ratio is about 2 to 4. In addition to being of narrow molecular weight distribution, the polyolefin of the invention is preferably semicrystalline. Desired polyolefins have a crystalline content of about 20 to 90, more desirably about 40 to 80%. The degree of crystallinity is linearly proportional to the density of the sample.

The container may further contain a small amount of an additional polymer, generally from about 0.1 % to 10% of an additional polymer. The additional polymer may be incorporated into the composition by copolymerization with the appropriate monomer. Such copolymers may be added to the composition to enhance other characteristics of the final composition, and may be, for example, polyacrylate, polyvinyl, polystyrene, etc.

Such polyolefin compositions include a radiation stabilizing additive, such as a mobilizing additive which contributes to the radiation stability of the container. Such a mobilizing additive may be a low molecular weight noncrystalline substance which is miscible with the polymeric material and is also compatible therewith, not adversely affecting the properties of the polymer. The mobilizer may be a substance which increases the free volume of the polymer and therefore, also lowers the density of the polymer, thereby increasing the radical termination reactions which prevent or minimize degradation during and subsequent to the irradiation. A wide variety of substances which increase the total free volume of the polymer may serve as the mobilizer, for example greases. Such mobilizers have a density from about 0.6 to 1.9 g/cm³ and a molecular weight being in the order from about 100 to 10,000 grams/mole. Examples for the mobilizing additive for the container include, but are not limited to hydrocarbon oils, halogenated hydrocarbon oils, phthalic ester oils, vegetable oils, mineral oils, silicone oils, and low molecular weight non-crystalline polymer greases. The mobilizing additive may be incorporated into the polymer in a mobilizing amount; generally about 0.1 to 50, desirably about 1 to 20% by weight.

Additionally or alternatively, the composition of the container may include a hindered amine stabilizer which contributes to the radiation stability of the container. Such a stabilizer may be a hindered amine which may be provided in the form of the free base, a salt, N-oxide, N-hydroxide or N-nitroxide thereof In these stabilizers, the nitrogen atom is part of a non-aromatic heterocyclic ring. The nitrogen is flanked by two carbon atoms, each bonded to two lower alkyl groups which may be the same or different, each lower alkyl group containing from 1 to 12 carbon atoms, or to an alicyclic group containing from 4 to 9 carbon atoms, which groups sterically hinder the amine. Preferred hindered amines for use in the compositions of the invention comprise a 5- or 6-membered heterocyclic ring containing the hindered amine nitrogen and optionally another hetero atom preferably nitrogen or oxygen. If the hindered amine is a tertiary amine, the tertiary group may be, for example, an optionally substituted alkyl, aralkyl, or alicyclic group containing from 1 to 12 carbon atoms. One or more of the substituents may be a hindered amine so that the tertiary group may be used to link a plurality of hindered amines. The hindering groups are preferably lower alkyl groups containing from 1 to 4 carbon atoms wherein, most preferably, all four groups are methyl. Preferred hindered amines are 2,2,4,4-tetramethyl piperidine derivatives.

Desirably, the hindered amine stabilizer is a hindered bis(4-piperidinyl)diester of a dicarboxylic acid. Representative examples of bis(hindered piperidinyl)diesters acceptable for use in the present invention, but not limited thereby, are the following: bis(2,2,6,6-tetramethyl-4-piperidinyl)sebacate; bis(1,2,2,6,6-pentamethyl-4-piperidinyl)-2-n-butyl-2-(3,5-di-tert-butyl-4- hydroxybenzyl) malonate; bis(1,2,2,6,6-pentamethyl-4-piperidinyl) sebacate, polymethylpropyl-3-oxy-[4(2,2,6,6-tetramethyl)piperidinyl]siloxane; poly[[6-[(1,1,3,3-tetramethylbutyl)amino]-1,3,5-triazine-2,4-diyl][(2,2,6,6-tetramethyl-4-piperidinyl) imino]-1,6-hexanediyl[(2,2,6,6-tetramethyl-4-piperidinyl)imino]]); and butanedioic acid dimethylester polymer with 4-hydroxy-2,2,6,6-tetramethyl-1-piperidine ethanol. The hindered amine stabilizer may be incorporated into the polymer in a radiation stabilizing amount. About 0.01 to 5.0, desirably about 0.05 to 3.0% by weight of the hindered amine stabilizer may be used.

In a particularly desirable embodiment, the container may be manufactured from a composition comprising a polyolefin having a crystalline content of about 20 to 90 percent and a weight distribution wherein the ratio of the weight average molecular weight to the number average molecular weight is no greater than 6, a mobilizing amount of a liquid mobilizer compatible with said polyolefin having a density of about 0.6 to 1.9 grams per cubic centimeter, and a radiation stabilizing amount of a hindered piperidine stabilizer.

As additional additives, the polyolefin composition may also contain a clarifier, such as a clarifying amount of a dibenzylidene sorbitol alkyl thioether clarifier. The container may further comprise a nucleating agent comprising a 2,2'-methylene-bis(4,6-di-t-butylphenol) phosphate salt such as sodium 2,2'-methylene-bis(4,6-di-t-butylphenol) phosphate or aluminum 2,2'-methylene-bis(4,6-di-t-butylphenol) phosphate. These and other additives may be provided in any useful amount, such as set forth in U.S. Patent Nos. 4,959,402 and 4,994,552.

The container is filled with a medium prior to being subjected to gamma irradiation for sterilization of the device. The medium is desirably a fluid material, and may be a therapeutic fluid or a non-therapeutic fluid, including materials such as such as a flush solutions, contrast agents, pharmaceutical agents and vaccines. Desirably, the medium is provided for parenteral administration to the human body, for example, saline water, or a pharmaceutical drug formulation.

As noted, medical devices prepared with radiation stable polyolefin compositions are known in the art, such as devices manufactured from COC or from the radiation stable compositions set forth in U.S. Patent Nos. 4,959,402 and 4,994,552. It is recognized through the present invention, however, that a synergy exists between the composition of the container, the type of radiation sterilization treatment, and whether a medium is present within the container.

For example, the use of a radiation stable polymer in a medical device may provide the device with stability with respect to physical characteristics of the polymer such as preventing weakening of the container, preventing leakage, and preventing yellowing. However, merely providing the device as being manufactured from a radiation stable polymer does not ensure protection of the contents of a container. For example, it has been discovered by the present inventors that gamma irradiation of an empty device which is manufactured of a radiation stable polymer followed by aseptically filling the device will not provide the contents of the device with a degree of safety and efficacy which meets recognized industry standards. In particular, when a syringe manufactured of a radiation stable polymer is subjected to ionizing radiation such as gamma radiation and then aseptically filled with a solution, the solution within that syringe degrades and does not maintain its physical properties, such as pH value and UV absorbance. Futhermore, the solution generates undesirable oxidizable matter, such as hydrogen peroxide, which can be harmful to the solution, particularly when used for parenteral administration.

Moreover, the present inventors have discovered that there is a marked difference in the properties of the contents of a device depending on the type of radiation treatment used in terminal sterilization procedures. For example, if E-beam radiation is used to terminally sterilize a prefilled syringe, a significant amount of oxidizable matter is produced within the solution contained within the syringe, which can render the solution unsafe for practical use.

The present inventors have recognized that a synergy exists between the container material, the contents of the container and the type of radiation treatment. Accordingly, in the present invention, adverse reaction of the contents of a prefilled container is inhibited during a radiation sterilization treatment by providing the container as a radiation stable polyolefin material, by prefilling the container with a medium prior to the radiation treatment, and by using gamma radiation for the radiation treatment. While not wishing to be bound by any particular theory, it is believed that prefilling the device appears to minimize the reactions incorporating radical scavengers in the container materials by providing a medium to neutralize the radical reactions during irradiation.

The gamma irradiation of the prefilled device may be conducted at any dosage useful to provide effective sterilization without degrading the device or its contents, using any known gamma radiation device. The amount of radiation depends on the amount of mass present. For a typical syringe type medical device, the gamma radiation is desirably provided at a range from about 10 kGy to about 60 kGy, more desirably from about 35 to 55 kGy.

The medical device may be irradiated at any point after filling. In particularly desirable embodiments, the medical device is packaged within a separate container or package such as a blister pack, as is known in the art. In such case, gamma irradiation may be conducted on the device after it has been contained within the final packaging, thereby providing for terminal sterilization after final packaging.

The present invention will be further exemplified through the following examples.

### EXAMPLES

### EXAMPLE 1

Example 1 sets forth a comparative example demonstrating terminal sterilization of a prefilled syringe using a conventional, non-radiation stable polyolefin polymer as the syringe material.

In particular, a set of 10 ml syringes sold under the name PROFAX PD 702 by Bassell Corp. of Elkton, Maryland were provided, manufactured of polypropylene which does not include any radiation stabilizing material therein.

As a reference, a set of fifty of these syringes were filled with a saline solution (VWR brand, available from VWR International, Inc., of Bridgeport, NJ).

Separately, a set of fifty of these syringes were prefilled with the same VWR saline solution, and then gamma irradiated by subjecting each of the prefilled syringes to gamma radiation at varying doses up to about 60 kGy using an IR 96 Co irradiator manufactured by MDS Dion of Canada.

Each of the reference syringes were tested for pH level, ultraviolet (UV) absorbance at 220-250 nm, and hydrogen peroxide (H₂O₂) content. Separately, each of the prefilled terminally sterilized syringes were also tested for similar properties. The results of the tests for the prefilled terminally sterilized syringes were averaged and compared with the results of the average for the reference syringes, with the change in values for each of the tested parameters shown below in Table 1.

**Table 1**

| **Gamma radiation Dose, kGy** | **ΔpH*¹*** | **ΔA*²* (220 - 350 nm)** | **Δ[H₂O₂]*³*, ppm** |
|---|---|---|---|
| 15 | - 0.99 | 0.10 | 1-3 |
| 20 | - 1.02 | 0.12 | 1-3 |
| 25 | - 1.05 | 0.12 | 1-3 |
| 30 | - 1.04 | 0.12 | 1 |
| 35 | - 0.98 | 0.08 | 1 |
| 40 | - 0.99 | 0.08 | 1 |
| 50 | - 0.98 | 0.06 | 1 |
| 60 | - 0.96 | 0.06 | 1 |

| | | | |
|---|---|---|---|
| *1* - change in value of pH level between average of reference samples and average of prefilled terminally sterilized samples *2* - change in value of UV absorbance between average of reference samples and average of prefilled terminally sterilized samples *3* - change in value of hydrogen peroxide content between average of reference samples and average of prefilled terminally sterilized samples | | | |

### EXAMPLE 2

Example 2 demonstrates the effects of terminal sterilization of a prefilled syringe using a radiation stable polyolefin polymer as the syringe material.

A set of 10 ml syringes manufactured of polyproylene and including a hindered piperidine stabilizer and mineral oil as a liquid mobilizer were provided, including a rubber stopper.

As a reference, a set of fifty of these radiation stable syringes were filled with the VWR saline solution as in Example 1. Separately, a set of fifty of these syringes were prefilled with the same VWR saline solution and terminally sterilized through gamma irradiation in the same manner as in Example 1. Each of the reference syringes and the terminally sterilized syringes were tested for pH level, ultraviolet (UV) absorbance at 220-250 and hydrogen peroxide (H₂O₂) content in a similar manner as in Example 1. The results of the tests for the prefilled terminally sterilized syringes were then averaged and compared with the results of the average for the reference syringes as in Example 1, with the change in values for each of the tested parameters shown below in Table 2.

**Table 2**

| **Gamma radiation Dose**, **kGy** | **ΔpH*¹*** | **ΔA*²* (220 - 350 nm)** | **Δ[H₂O₂]*³*, ppm** |
|---|---|---|---|
| 20 | - 0.63 | -0.01 | 0.13 |
| 25 | - 0.55 | -0.01 | 0.11 |
| 35 | - 0.63 | -0.01 | 0.11 |
| 45 | - 0.64 | 0.00 | 0.11 |
| 55 | - 0.70 | 0.00 | 0.10 |

| | | | |
|---|---|---|---|
| *1* - change in value of pH level between average of reference samples and average of prefilled terminally sterilized samples *2* - change in value of UV absorbance between average of reference samples and average of prefilled terminally sterilized samples *3* - change in value of hydrogen peroxide content between average of reference samples and average of prefilled terminally sterilized samples | | | |

A comparison of the results of Examples 1 and 2 as shown in Tables 1 and 2 demonstrates that the degradation of the samples prepared in accordance with the present invention was significantly lower than the samples of the prior art. In particular, the samples prepared according to Example 1, in which a non-radiation stable polypropylene syringe is prefilled and then terminally sterilized, reflect a drastic change in pH value, UV absorbance and hydrogen peroxide content when compared with the reference samples, over a variety of gamma radiation doses from 15 kGy to 60 kGy. On the other hand, the samples prepared in accordance with the present invention, in which a radiation stable polyolefin syringe is prefilled and then terminally sterilized, reflect only minor changes in the same properties when compared with the reference values. Accordingly, the quality of the contents of the syringe based on the change in pH and UV absorbance is significantly increased, and extremely low levels of oxidizing material such as hydrogen peroxide are present when radiation stabilized polyolefins are used for the terminal sterilization method as opposed to non-radiation stabilized polyolefins.

### EXAMPLE 3

Example 3 demonstrates the difference in properties between radiation stable syringes which are assembled, sterilized and then aseptically filled, as compared with radiation stable syringes which are assembled and prefilled followed by terminal sterilization. In particular, a set of syringes such as those from Example 2, manufactured of polyproylene and including a hindered piperidine stabilizer and mineral oil as a liquid mobilizer, were provided in both 3 ml and 10 ml sizes.

As a reference set A, fifty of the syringes were filled with the VWR saline solution and stored at 70°C overnight.

Separately, fifty of the syringes identified as set B were gamma irradiated while empty by subjecting each of the empty syringes to gamma radiation at a dose of 45 kGy using the irradiator of Example 1. After gamma irradiation, each of the empty syringes were filled with the VWR saline solution under aseptic conditions in a clean room environment. Each of the irradiated then aseptically filled syringes of set B were stored at 70°C overnight.

A further set of fifty of the syringes identified as set C were filled with the VWR saline solution, and then terminally sterilized by individually subjecting each of the prefilled syringes to gamma radiation at a dose of 45 kGy. These prefilled terminally irradiated syringes of set C were also stored at 70°C overnight.

Sets of the sample syringes were prepared for both 3 ml size and 10 ml size syringes.

After overnight storage, all of the reference syringes of set A, the irradiated and aseptically filled syringes of set B, and the prefilled terminally irradiated syringes of set C were tested for pH level and UV absorbance at 220-250 nm after storage overnight at 70°C, after storage for 2 weeks at 40°C, and after storage for 2 weeks at 40°C followed by storage for 1 year at room temperature (23°C). The results of the tests for the irradiated and aseptically filled syringes of set B and the prefilled terminally irradiated syringes of set C were averaged and compared with the results of the averages for the reference syringes of set A, with the change in values for each of the tested parameters shown below in Table 3.

**Table 3**

| | **Time** | **3 ml Size Syringe** | | **10 ml Size Syringe** | |
|---|---|---|---|---|---|
| | | **Set B** | **Set C** | **Set B** | **Set C** |
| **ΔpH*¹*** | 0*³* | -1.98 | - 0.73 | -1.78 | - 0.78 |
| | 2 wks @ 40 °C | -1.83 | - 0.53 | -1.71 | - 0.54 |
| | 2 wks @ 40°C + 1 yr @ RT | -1.83 | -0.23 | | |
| **ΔA*²*** (220 - 350 nm) | 0*³* | 0.099 | 0.028 | 0.056 | 0.015 |
| | 2 wks @ 40°C | 0.063 | 0.018 | 0.044 | 0.011 |
| | 2 wks @ 40°C + 1 yr @ RT | 0.051 | 0.010 | | |

| | | | | | |
|---|---|---|---|---|---|
| *1 -* change in value of pH level between average of reference samples and average of aseptically filled samples 2 - change in value of UV absorbance between average of reference samples and average of aseptically filled samples 3 - time 0 represents after overnight at 70°C | | | | | |

A comparison of the results of Example 3 as shown in Table 3 unexpectedly demonstrates the decrease in degradation of the sample within the syringe when the syringe is prefilled with a sample prior to gamma radiation as a terminal sterilization treatment as opposed to gamma irradiation prior to aseptic fill, even when a radiation stable polymer is used as the syringe material. In particular, the samples of set B, in which a radiation stable polypropylene syringe is first gamma irradiated and then aseptically filled, reflect a drastic change in pH value and UV absorbance when compared with the reference samples, whether at time zero or after prolonged storage. On the other hand, the samples of set C, prepared in accordance with the present invention in which a radiation stable polyolefin syringe is prefilled and then terminally sterilized with gamma radiation demonstrate significantly less change in the pH and UV absorbance when compared with the reference values. As such, by prefilling and then gamma irradiating the syringes, the samples contained therein unexpectedly demonstrate marked improvement in the quality of the sample when compared with aseptically filled gamma irradiated syringes.

### EXAMPLE 4

Example 4 compares the effects of terminal sterilization of prefilled syringes through the use of gamma radiation and E-beam radiation. In particular, two syringes manufactured of a radiation stable polypropylene material as set forth in Example 2 were separately filled with the VWR saline solution as discussed above, and labeled as Syringes D and E. After filling, Syringe D was subjected to E-beam radiation at a dose of 5.6 Mrad, while syringe E was subjected to gamma radiation at a dose of 4.0 Mrad.

Further, two syringes manufactured of a cyclic olefin copolymer (COC) were separately filled with the VWR saline solution, and labeled as Syringes F and G. After filling, Syringe F was subjected to E-beam radiation at a dose of 5.6 Mrad, while syringe G was subjected to gamma radiation at a dose of 4.0 Mrad.

The contents of each of Syringes D-G was thereafter evaluated for hydrogen peroxide concentration, with the results shown in Table 4.

**Table 4**

| | **Syringe D** **(polypropylene, E-beam)** | **Syringe E** **(polypropylene, gamma)** | **Syringe F** **(COC, E-beam)** | **Syringe G** **(COC, gamma)** |
|---|---|---|---|---|
| **[H₂O₂], ppm** | 3 | 0.1 | 2 | 0.1 |

A comparison of the results of Example 4 as shown in Table 4 demonstrates that the amount of oxidizable material within a sample contained in a radiation stable polymeric device is greatly reduced by prefilling the syringe and subjecting it to gamma irradiation as opposed to E-beam radiation. Such results hold true regardless of the nature of the radiation stable polymer.

The present invention has been described with reference to specific details of particular embodiments thereof. It is not intended that such details be regarded as limitations upon the scope of the invention except insofar as and to the extent that they are included in the accompanying claims.
1. A method for inhibiting adverse reaction of the contents of a prefilled container during a radiation sterilization procedure comprising:
   providing the container made of a radiation stable polyolefin material; and
   prefilling the container with a medium prior to subjecting the container to a gamma irradiation sterilization treatment.
2. A method as in 1, wherein the medium is selected from the group consisting of a therapeutic fluid and a non-therapeutic fluid.
3. A method as in 2, wherein the medium comprises a drug for parenteral administration to the body.
4. A method as in 2, wherein the medium comprises saline water.
5. A method as in 1, wherein the medium has a pH between about 4.5 and about 7.0 after radiation sterilization.
6. A method as in 1, wherein the medium exhibits ultraviolet absorbance of less than about 0.2 at a wavelength between 220 and 340 nm.
7. A method as in 1, wherein the medium includes less than about 3.4 ppm of hydrogen peroxide.
8. A method as in 1, wherein the container is manufactured from a composition comprising a polyolefin, a mobilizing amount of a liquid mobilizer compatible with said polyolefin, and a radiation stabilizing amount of a hindered piperidine stabilizer.
9. A method as in 8, wherein the composition of the container further comprises a clarifying amount of a dibenzylidene sorbitol alkyl thioether clarifier.
10. A method as in 8, wherein the composition of the container further comprises a nucleating agent comprising a 2,2'-methylene-bis(4,6-di-t-butylphenol)phosphate salt.
11. A method as in 10, wherein the 2,2'-methylene-bis(4,6-di-t-butylphenol)phosphate salt is selected from the group consisting of sodium 2,2'-methylene-bis(4,6-di-t-butylphenol)phosphate and aluminum 2,2'-methylene-bis(4,6-di-t-butylphenol) phosphate.
12. A method as in 8, wherein the composition of the container further comprises about 0.1 to 10% of an additional polymer.
13. A method as in 8, wherein the polyolefin is selected from the group consisting of polyethylene, polypropylene, polymethylpentene, polytetrafluoroethylene and copolymers thereof.
14. A method as in 8, wherein the mobilizing additive is selected from the group consisting of a hydrocarbon oil, phthalic ester oil, polymer grease, vegetable oil, mineral oil and silicone oil.
15. A method as in 8, wherein the stabilizer is a bis(4-piperidinyl) diester of a dicarboxylic acid.
16. A method as in 1, where the gamma irradiation ranges from about 10 kGy to about 60 kGy.
17. A method of sterilizing a prefilled container comprising:
   providing a container made of a radiation stable polyolefin material;
   filling the container with a medium; and
   irradiating said container filled with said medium with gamma radiation.
18. A method as in 17, further comprising a step of sealing the container after filling the container with the medium and prior to irradiating the container.
19. A method as in 18, further comprising a step of enclosing the container within packaging after sealing the container, and wherein the irradiating step comprises irradiating said container within said packaging.
20. A method as in 19, wherein said packaging comprises a blister package.
21. A method as in 17, wherein the medium is selected from the group consisting of a therapeutic fluid and a non-therapeutic fluid.
22. A method as in 17, wherein the medium comprises a drug for parenteral administration to the body.
23. A method as in 17, wherein the medium comprises a saline water.
24. A method as in 17, wherein the gamma radiation is in a range from about 10 kGy to about 60 kGy.
25. A method as in 17, wherein the container is manufactured from a composition comprising a polyolefin, a mobilizing amount of a liquid mobilizer compatible with said polyolefin, and a radiation stabilizing amount of a hindered piperidine stabilizer.
26. A method as in 25, wherein the container further comprises a clarifying amount of a dibenzylidene sorbitol alkyl thioether clarifier.
27. A method as in 25, wherein the container further comprises a nucleating agent comprising a 2,2'-methylene-bis(4,6-di-t butylphenol)phosphate salt.
28. A method as in 25, wherein the composition of the container further comprises about 0.1 to 10% of an additional polymer.
29. A method as in 25, wherein the polyolefin is selected from the group consisting of polyethylene, polypropylene, polymethylpentene, polytetrafluoroethylene and copolymers thereof.
30. A method as in 25, wherein the container comprises a bag for intravenous fluid delivery.
31. A method as in 25, wherein the container comprises a syringe.
32. A sterilized article comprising:
   a container made of a radiation stable polyolefin material; and
   a medium contained within said container,
   wherein said container containing said medium therein has been subjected to a gamma irradiation sterilization treatment after being filled with said medium.
33. A sterilized article as in 32, wherein the medium is selected from the group consisting of a therapeutic fluid and a non-therapeutic fluid.
34. A sterilized article as in 32, wherein the medium contained within the container comprises a drug for parenteral administration to the body.
35. A sterilized article as in 32, wherein the medium comprises saline water.
36. A sterilized article as in 32, wherein the container comprises a bag for intravenous fluid delivery.
37. A sterilized article as in 32, wherein the container comprises a syringe.
38. A sterilized article as in 32, wherein the container is manufactured from a composition comprising a polyolefin, a mobilizing amount of a liquid mobilizer compatible with said polyolefin, and a radiation stabilizing amount of a hindered piperidine stabilizer.
39. A sterilized article as in 38, wherein the container further comprises a clarifying amount of a dibenzylidene sorbitol alkyl thioether clarifier.
40. A sterilized article as in 38, wherein the container further comprises a nucleating agent comprising a 2,2'-methylene-bis(4,6-di-t-butylphenol)phosphate salt.
41. A sterilized article as in 40, wherein the 2,2'-methylene-bis(4,6-di-t-butylphenol)phosphate salt is selected from the group consisting of sodium 2,2'-methylene-bis(4,6-di-t-butylphenol)phosphate and aluminum 2,2'-methylene-bis(4,6-di-t-butylphenol)phosphate.
42. A sterilized article as in 38, wherein the composition of the container further comprises about 0.1 to 10% of an additional polymer.
43. A sterilized article as in 38, wherein the polyolefin is selected from the group consisting of polyethylene, polypropylene, polymethylpentene, polytetrafluoroethylene and copolymers thereof
44. A sterilized article as in 38, wherein the mobilizing additive is selected from the group consisting of a hydrocarbon oil, phthalic ester oil, polymer grease, vegetable oil, mineral oil and silicone oil.
45. A sterilized article as in 38, wherein the stabilizer is a bis(4-piperidinyl) diester of a dicarboxylic acid.

## Claims

1. A sterilized article comprising:
a container made of a radiation stable polyolefin material; and
a medium contained within said container,
wherein said container containing said medium therein has been subjected to a gamma irradiation sterilization treatment after being filled with said medium.

2. A sterilized article as in claim 1, wherein the medium is selected from the group consisting of a therapeutic fluid and a non-therapeutic fluid.

3. A sterilized article as in claim 1, wherein the medium contained within the container comprises a drug for parenteral administration to the body.

4. A sterilized article as in claim 1, wherein the medium comprises saline water.

5. A sterilized article as in claim 1, wherein the container comprises a bag for intravenous fluid delivery.

6. A sterilized article as in claim 1, wherein the container comprises a syringe.

7. A sterilized article as in claim 1, wherein the container is manufactured from a composition comprising a polyolefin, a mobilizing amount of a liquid mobilizer compatible with said polyolefin, and a radiation stabilizing amount of a hindered piperidine stabilizer.

8. A sterilized article as in claim 7, wherein the container further comprises a clarifying amount of a dibenzylidene sorbitol alkyl thioether clarifier.

9. A sterilized article as in claim 7, wherein the container further comprises a nucleating agent comprising a 2,2'-methylene-bis(4,6-di-t-butylphenol)phosphate salt.

10. A sterilized article as in claim 9, wherein the 2,2'-methylene-bis(4,6-dit-butylphenol)phosphate salt is selected from the group consisting of sodium 2,2'-methylene-bis(4,6-di-t-butylphenol)phosphate and aluminum 2,2'-methylene-bis(4,6-di-t-butylphenol)phosphate.

11. A sterilized article as in claim 7, wherein the composition of the container further comprises about 0.1 to 10% of an additional polymer.

12. A sterilized article as in claim 7, wherein the polyolefin is selected from the group consisting of polyethylene, polypropylene, polymethylpentene, polytetrafluoroethylene, and copolymers thereof.

13. A sterilized article as in claim 7, wherein the mobilizing additive is selected from the group consisting of a hydrocarbon oil, phthalic ester oil, polymer grease, vegetable oil, mineral oil, and silicone oil.

14. A sterilized article as in claim 7, wherein the stabilizer is a bis(4-piperidinyl) diester of a dicarboxylic acid.
